# FASCICULE DE BREVET EUROPEEN

(11) **EP 4 514 299 B1**
(45) Date de publication et mention de la délivrance du brevet: **21.01.2026**
(21) Numéro de dépôt: 23722307.8
(22) Date de dépôt: 25.04.2023
(51) Int. Cl.: A61H 1/02, B25J 9/00, A61F 5/01

(54) **ORTHÈSE COMPRENANT UNE LIAISON MÉCANIQUE DE HANCHE À TROIS DEGRÉS DE LIBERTÉ**
ORTHESE MIT EINER MECHANISCHEN HÜFTGELENKVERBINDUNG MIT DREI FREIHEITSGRADEN
ORTHOSIS COMPRISING A MECHANICAL HIP CONNECTION WITH THREE DEGREES OF FREEDOM

(30) Priorité: 27.04.2022 FR 2203952
(43) Date de publication de la demande: 05.03.2025
(73) Titulaire: Wandercraft, 75004 Paris (FR)
(72) Inventeur: D'HIVER, Yoann, 92120 MONTROUGE (FR); BECK, Maxime, 92110 CLICHY (FR); DONAT, Malo, 75005 PARIS (FR)
(74) Mandataire: Regimbeau
(86) Numéro de dépôt international: PCT/EP2023/060765
(87) Numéro de publication internationale: WO 2023/208904

(56) Documents cités:
- FR-A1- 3 086 709
- US-A1- 2017 143 573

## Description

### DOMAINE TECHNIQUE

La présente demande concerne un système d'aide à la mobilité d'une personne, ou exosquelette, capable notamment de supporter un utilisateur touché par une déficience motrice.

### ETAT DE LA TECHNIQUE

Un exosquelette comprend, de manière générale, une structure de bassin, deux structures de jambes, deux structures de pied et deux structures de hanche. La structure de bassin est configurée pour se positionner derrière les reins d'un utilisateur lorsqu'il porte l'exosquelette et peut être attachée au bassin à l'aide d'un harnais ou de sangles au corps de l'utilisation. Chaque structure de jambe est configurée pour se positionner en regard de l'une des jambes (gauche ou droite, selon la structure) de l'utilisateur, et comprend un segment de jambe supérieur et un segment de jambe inférieur, agencés pour venir en regard de la cuisse et du mollet de l'utilisateur, respectivement. Chaque structure de pied comprend par ailleurs un plan de support sur lequel l'un des pieds (gauche ou droit, selon la structure) de l'utilisateur peut venir en appui lorsque le pied est à plat. Enfin, chaque structure de hanche est configurée pour se positionner en regard de l'une des hanches (gauche ou droite, selon la structure).

Le contrôle complet de l'exosquelette nécessite des actionneurs et des liaisons mécaniques pour permettre le mouvement de l'exosquelette et autoriser ainsi un déplacement de l'utilisateur portant l'exosquelette. Les liaisons mécaniques comprennent généralement des liaisons pivot, glissière et/ou rotule, tandis que les actionneurs peuvent comprendre notamment des vérins ou des moteurs.

Ces liaisons mécaniques et actionneurs sont choisis de manière à permettre le déplacement de l'exosquelette sans risquer de blesser l'utilisateur qui le porte. A cet effet, il est notamment important de ne pas appliquer d'efforts non supportés par les membres de l'utilisateur et de proposer un exosquelette présentant à la fois un faible encombrement et un poids modéré.

Afin de permettre le mouvement de marche, l'exosquelette comprend notamment des liaisons mécaniques de hanche configurées pour relier la structure de hanche et chaque structure de jambe. Généralement, ces liaisons mécaniques comprennent une liaison pivot configurée pour permettre des mouvements de flexion/extension dans le plan sagittal de l'utilisateur. Certains exosquelettes peuvent comprendre une liaison pivot additionnelle configurée pour permettre des mouvements d'abduction/adduction dans le plan frontal. Les axes de pivotement de ces liaisons mécaniques sont proches des axes de pivotement de la hanche de l'utilisateur et les liaisons pivot sont montées sur l'exosquelette de sorte à s'étendre à la périphérie du corps de l'utilisateur. L'utilisateur peut ainsi se déplacer vers l'avant ou l'arrière dans l'exosquelette.

Certains exosquelettes comme l'exosquelette Atalante commercialisé par la Demanderesse comprennent en outre une troisième liaison pivot configurée pour permettre des mouvements de rotation interne/externe dans le plan transversal de l'utilisateur. Toutefois, pour reproduire la liaison pivot transversale de l'être humain, cette liaison pivot devrait être positionnée au sein de l'exosquelette, à l'endroit où se trouve le patient. La troisième liaison pivot de l'exosquelette Atalante a donc été déportée sur la partie arrière de l'exosquelette. Toutefois, ce déport a pour conséquence de générer des mouvements parasites de l'utilisateur (son bassin devant avancer lors des mouvements de rotation externe) et implique en outre de limiter le débattement de la structure de jambe par rapport à la structure de hanche à une vingtaine de degrés dans le plan transversal pour éviter tout risque de blessure de l'utilisateur résultant de ces mouvements parasites. Les mouvements possibles de l'exosquelette, et donc de l'utilisateur, bien qu'améliorés en comparaison avec les liaisons mécaniques dépourvues de liaison transversale, peuvent encore être optimisés.

Le document US 2017/143573 décrit un exosquelette comprenant une liaison mécanique de hanche.

### EXPOSE DE L'INVENTION

Un but de la présente demande est de remédier aux inconvénients précités, en proposant une orthèse pour un être vivant, de préférence un être humain, comprenant une liaison mécanique de hanche capable de permettre davantage de mouvements dans les plans frontal, sagittal et transversal, qui soit plus ergonomique et plus anthropomorphe que les orthèses connues.

Il est à cet effet proposé, selon un premier aspect, une orthèse comprenant une liaison mécanique de hanche reliant une structure de hanche configurée pour se positionner en regard d'une hanche d'un utilisateur et une structure de jambe configurée pour recevoir une jambe de l'utilisateur, la liaison mécanique de hanche comprenant une liaison pivot frontale configurée pour autoriser un mouvement de rotation de la structure de jambe par rapport à la structure de hanche selon un axe de pivotement frontal de l'orthèse, une liaison pivot sagittale configurée pour autoriser un mouvement de rotation de la structure de jambe par rapport à la structure de hanche selon un axe de pivotement sagittal de l'orthèse et une liaison pivot transversale configurée pour autoriser un mouvement de rotation de la structure de jambe par rapport à la structure de hanche selon un axe de pivotement transversal de l'orthèse. De plus, l'axe de pivotement sagittal et l'axe de pivotement frontal étant concourants. Par ailleurs, la liaison pivot transversale étant composée d'une pluralité de liaisons pivot raccordées deux à deux par des biellettes de sorte à former une liaison pivot virtuelle de sorte que l'axe de pivotement transversal coupe le volume délimité par le plan sagittal, par un plan normal à l'axe de pivotement frontal et comprenant l'axe de pivotement sagittal, par la structure de hanche et par la structure de jambe.

Certaines caractéristiques préférées mais non limitatives de l'orthèse selon le premier aspect sont les suivantes, prises individuellement ou en combinaison :
- l'axe de pivotement transversal est normal à un plan formé par l'axe de pivotement frontal et l'axe de pivotement sagittal, un premier point d'intersection défini par l'intersection entre le plan et l'axe de pivotement transversal étant situé à une distance inférieure à 150 mm d'un deuxième point d'intersection défini par l'intersection des axes de pivotement sagittal et frontal, de préférence inférieure à 120 mm, de préférence inférieure ou égale à 100 mm, par exemple inférieure ou égale à 92 mm ;
- le premier point d'intersection est situé à une distance inférieure ou égale à 90 mm du plan sagittal de l'orthèse, de préférence inférieure ou égal à 60 mm, plus préférentiellement inférieure ou égale à 40 mm, par exemple supérieure ou égale à 0 mm et inférieure ou égale à 10 mm ;
- le premier point d'intersection est situé à une distance inférieure ou égale à 90 mm d'un plan parallèle au plan frontal de l'orthèse et comprenant l'axe de pivotement sagittal, de préférence inférieure ou égale à 70 mm ;
- les liaisons pivot composant la liaison pivot transversale comprennent :
   * deux premières liaisons pivot raccordées par une première biellette ;
   * une deuxième et une troisième liaison pivot raccordées chacune à une première liaison pivot correspondante par une deuxième et une troisième biellette, respectivement, la deuxième et la troisième biellette étant de même longueur ;
   * une quatrième liaison pivot raccordée à la deuxième liaison pivot par une quatrième biellette qui est encastrée avec la deuxième biellette ;
   * une cinquième et une sixième liaison pivot raccordées respectivement à la deuxième liaison pivot et à la quatrième liaison pivot par une cinquième et une sixième biellette, respectivement, la cinquième et la sixième biellette étant de même longueur;
   * une septième biellette raccordant la deuxième et la troisième liaison pivot et étant encastrée avec la cinquième biellette ; et
   * une huitième biellette raccordant la cinquième et la sixième liaison pivot ;
- la liaison pivot frontale est raccordée mécaniquement à la structure de hanche, la liaison pivot sagittale est raccordée mécaniquement à la structure de jambe, et la liaison pivot transversale est raccordée mécaniquement à la liaison pivot frontale et à la liaison pivot sagittale ;
- l'orthèse comprend en outre un actionneur transversal configuré pour actionner la liaison pivot transversale, un actionneur frontal configuré pour actionner la liaison pivot frontale et un actionneur sagittal configuré pour actionner la liaison pivot sagittale, l'actionneur transversal, l'actionneur frontal et l'actionneur sagittal étant indépendants les uns des autres ;
- l'orthèse comprend en outre en outre un actionneur transversal configuré pour actionner la liaison pivot transversale, l'actionneur transversal comprenant une biellette d'actionnement comportant une première extrémité raccordée à la liaison pivot transversale par l'intermédiaire d'une première liaison rotule et une deuxième extrémité raccordée à la structure de hanche par l'intermédiaire d'une deuxième liaison rotule ;
- la deuxième liaison rotule est décalée par rapport à l'axe de pivotement frontal ;
- l'actionneur transversal comprend une platine d'actionnement coaxiale à l'axe de pivotement frontal ;
- la deuxième liaison rotule est raccordée à la platine d'actionnement en étant décalée par rapport à l'axe de pivotement frontal ;
- l'axe de pivotement sagittal de la liaison pivot sagittale est solidaire en mouvement d'un centre de rotation de la première liaison rotule ;
- l'orthèse comprend en outre en outre un actionneur frontal configuré pour actionner la liaison pivot frontale, l'actionneur frontal étant déporté par rapport à l'axe de pivotement frontal ;
- l'actionneur frontal comprend deux câbles montés croisés d'une part sur un arbre de sortie de l'actionneur frontal et d'autre part sur la liaison pivot frontale afin de faire pivoter la liaison pivot frontale autour de l'axe de pivotement frontal ;
- l'orthèse comprend en outre en outre au moins l'une parmi les butées mécaniques suivantes : une butée frontale configurée pour limiter une rotation de la structure de jambe autour de l'axe de pivotement frontal, une butée sagittale configurée pour limiter une rotation de la structure de jambe autour de l'axe de pivotement sagittal, une butée transversale configurée pour limiter une rotation de la structure de jambe autour de l'axe de pivotement transversal ; et/ou
- l'orthèse est un exosquelette.

L'invention s'applique par exemple à des exosquelettes, mais également à tout type d'orthèse comprenant une structure de hanche et une structure de jambe.

### DESCRIPTION DES FIGURES

D'autres caractéristiques, buts et avantages de l'invention ressortiront de la description qui suit, qui est purement illustrative et non limitative, et qui doit être lue en regard des dessins annexés sur lesquels :
La figure 1 est une modélisation cinématique d'un exemple de réalisation d'une liaison mécanique de hanche pour une orthèse conforme à un mode de réalisation de l'invention, lorsque l'orthèse est au repos ;
La figure 2 est une vue de dessus de la modélisation cinématique de la figure 1 ;
La figure 3 correspond à la modélisation cinématique de la figure 1 lors d'un mouvement de rotation pure autour de l'axe de pivotement frontal de l'orthèse ;
La figure 4 correspond à la modélisation cinématique de la figure 1 lors d'un mouvement de rotation pure autour de l'axe de pivotement transversal de l'orthèse ;
La figure 5 correspond à la modélisation cinématique de la figure 1 lors d'un mouvement de rotation pure autour de l'axe de pivotement sagittal de l'orthèse ;
La figure 6 est une vue simplifiée en perspective d'un mode de réalisation d'une orthèse conforme à un mode de réalisation de l'invention illustrant un exemple d'actionnement de la liaison mécanique transversale de l'orthèse ;
La figure 7 est une vue de dos simplifiée d'un exemple de réalisation d'une orthèse conforme à un mode de réalisation de l'invention illustrant un exemple d'actionnement de la liaison mécanique frontale de l'orthèse, la structure de jambe étant illustrée en deux positions différentes ;

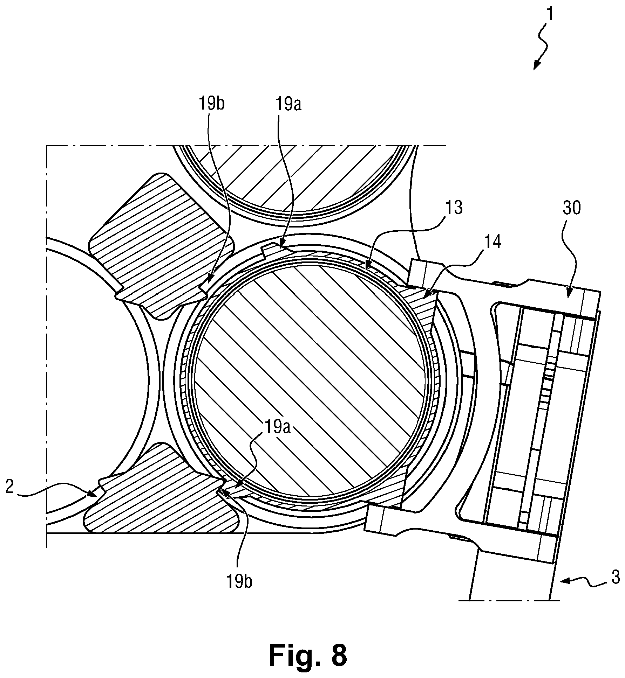

La figure 8 est une vue de dos simplifiée d'un exemple de réalisation d'une orthèse conforme à un mode de réalisation de l'invention illustrant un exemple de butées mécaniques dans la liaison pivot frontale ;
La figure 9 est une vue de dessus simplifiée d'un exemple de réalisation d'une orthèse conforme à un mode de réalisation de l'invention illustrant un exemple de butée mécanique dans la liaison pivot transversale ;
La figure 10 est une vue de côté simplifiée d'un exemple de réalisation d'une orthèse conforme à un mode de réalisation de l'invention illustrant un exemple de butées mécaniques dans la liaison pivot sagittale ; et

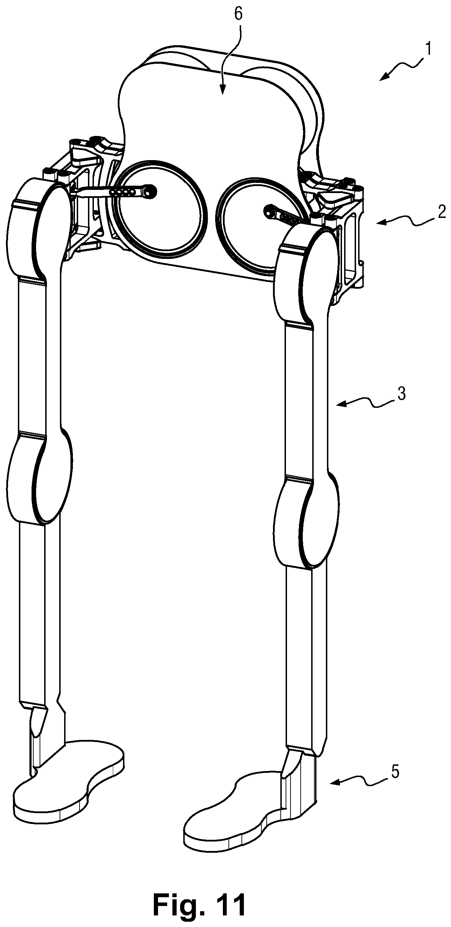

La figure 11 est une vue simplifiée d'un exemple d'exosquelette comprenant une liaison mécanique de hanche conforme à un mode de réalisation de l'invention.

Sur l'ensemble des figures, les éléments similaires portent des références identiques.

### DESCRIPTION DETAILLEE DE L'INVENTION

Une orthèse 1 conforme à l'invention comprend une structure de hanche 2 configurée pour se positionner en regard d'une hanche d'un utilisateur et une structure de jambe 3 configurée pour recevoir une jambe de l'utilisateur. La structure de hanche 2 et la structure de jambe 3 sont configurées pour être raccordées par une liaison mécanique de hanche 4.

La structure de jambe 3 comprend une structure de jambe 3 supérieure configurée pour recevoir une cuisse d'un utilisateur, et optionnellement une structure de jambe 3 inférieure configurée pour recevoir un mollet de l'utilisateur. Le cas échéant, la structure de jambe 3 inférieure et la structure de jambe 3 supérieure sont raccordées par une liaison mécanique de genou permettant le mouvement de la structure de jambe 3 inférieure par rapport à la structure de jambe 3 supérieure. La structure de hanche 2 quant à elle s'étend latéralement par rapport à la hanche correspondante de l'utilisateur.

Dans ce qui suit, l'invention sera décrite dans le cas d'une orthèse 1 comprenant la structure de hanche 2 et une structure de jambe 3. L'invention s'applique cependant à toute orthèse 1 pour le corps humain ou animal comprenant une structure de hanche 2 et une structure de jambe 3, par exemple à une orthèse 1 comprenant une structure de jambe 3 droite et une structure de jambe 3 gauche, avec ou sans structure de jambe 3 inférieure, et le cas échéant avec une structure de pied 5 raccordée par une liaison mécanique de cheville chaque structure de jambe 3 inférieure. L'invention s'applique alors de manière symétrique à la liaison mécanique raccordant la structure de hanche 2 à la structure de jambe 3 droite et à la structure de jambe 3 gauche. Optionnellement, l'orthèse 1 peut également comprendre une structure de bassin 6, configurée pour se positionner derrière les reins de l'utilisateur lorsqu'il porte l'orthèse 1 et qui peut être attachée au bassin de l'utilisateur à l'aide d'un harnais ou de sangles.

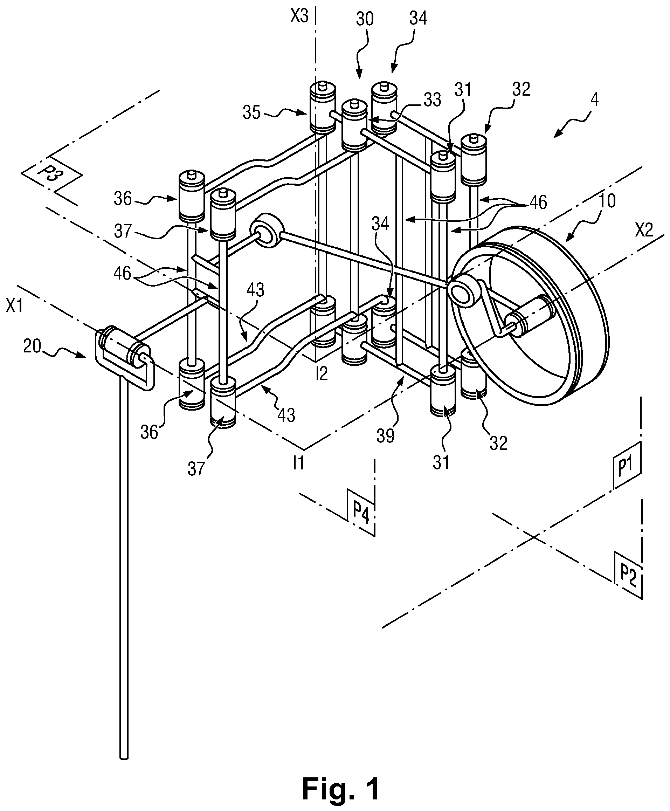

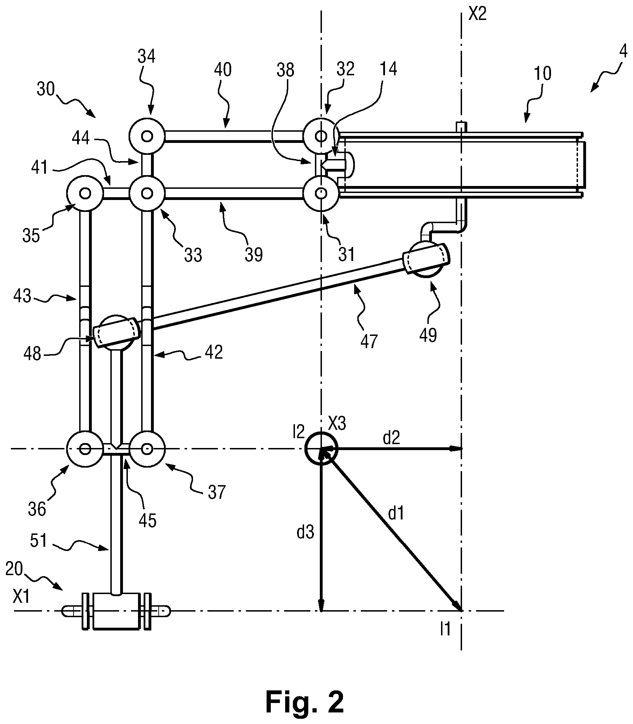

L'orthèse 1 sera décrite dans ce qui suit en relation avec des plans et axes de référence, et plus particulièrement un plan sagittal P1, un plan transversal P3 et un plan frontal P2 de l'orthèse 1. La direction d'extension de ces plans est définie lorsque l'orthèse 1 est portée par un utilisateur qui se tient debout, droit, les pieds serrés et parallèles entre eux. Dans ce qui suit, cette position sera définie comme la position au repos de l'orthèse 1 (ou position neutre - voir figures 1 et 2).

Lorsque l'orthèse 1 comprend deux structures de jambe, le plan sagittal P1 correspond au plan de symétrie verticale de l'orthèse 1, qui passe par le centre de la structure de hanche 2. La structure de jambe 3 droite est alors sensiblement symétrique à la structure de jambe 3 gauche par rapport au plan sagittal P1. On notera que le plan sagittal P1 d'une orthèse 1 conforme à l'invention et ne comprenant qu'une seule structure de jambe 3 est positionné au même endroit de l'orthèse 1 que si l'orthèse 1 comprenait deux structures de jambe. C'est donc dans ce plan sagittal P1 qu'ont lieu les mouvements de flexion et d'extension de la structure de jambe 3 par rapport à la structure de hanche 2 liés par exemple à la marche.

Le plan frontal P2 de l'orthèse 1 est normal au plan sagittal P1 et divise l'orthèse 1 en deux parties, une partie antérieure (ventrale) et une partie postérieure (dorsale). C'est dans ce plan qu'ont lieu les mouvements d'abduction et d'adduction de la structure de jambe 3 par rapport à la structure de hanche 2 liés par exemple à des mouvements de déhanchement.

Le plan transversal P3 de l'orthèse 1 est normal au plan sagittal P1 et au plan frontal P2. Il est parallèle au sol et divise l'orthèse 1 en deux parties, une partie supérieure (du côté du bassin de l'utilisateur) et une partie inférieure (du côté des pieds de l'utilisateur). C'est dans ce plan qu'ont lieu les mouvements de rotation interne et externe de la structure de jambe 3 par rapport à la structure de hanche 2, liés par exemple à des mouvements de torsion de la structure de jambe 3.

La liaison mécanique de hanche 4 comprend :
- une liaison pivot frontale 10 configurée pour autoriser un mouvement de rotation de la structure de jambe 3 par rapport à la structure de hanche 2 autour d'un axe de pivotement frontal X2 qui est normal au plan frontal P2 de l'orthèse 1 ;
- une liaison pivot sagittale 20 configurée pour autoriser un mouvement de rotation de la structure de jambe 3 par rapport à la structure de hanche 2 autour d'un axe de pivotement sagittal X1 qui est normal au plan sagittal P1 de l'orthèse 1 lorsque l'orthèse 1 est au repos ; et
- une liaison pivot transversale 30 configurée pour autoriser un mouvement de rotation de la structure de jambe 3 par rapport à la structure de hanche 2 autour d'un axe de pivotement transversal X3 qui est normal au plan transversal P3 de l'orthèse 1 lorsque l'orthèse 1 est au repos.

L'axe de pivotement sagittal X1 et l'axe de pivotement frontal X2 sont concourants, c'est-à-dire qu'ils se croisent au niveau d'un premier point d'intersection 11. De préférence, lorsqu'un utilisateur porte l'orthèse 1, au repos, ce premier point d'intersection I1 est confondu avec le centre de rotation de l'articulation de la hanche correspondante de l'utilisateur (articulation coxo-fémorale qui est assimilable à une liaison rotule sphérique).

La liaison pivot transversale 30 est composée d'une pluralité de liaisons pivot 31-37 raccordées deux à deux par des biellettes 38-45 de sorte à former une liaison pivot virtuelle 30 de sorte que l'axe de pivotement transversal X3 coupe le volume délimité par le plan sagittal P1, par un plan P4 normal à l'axe de pivotement frontal X2 et comprenant l'axe de pivotement sagittal X1, par la structure de hanche 2 et par la structure de jambe 3. En d'autres termes, l'intersection l2 entre l'axe de pivotement (transversal) de la liaison pivot virtuelle 30 et le plan comprenant les axes de pivotement frontal X2 et sagittal X1 est situé dans le volume qui s'étend entre la structure de hanche 2 et la structure de jambe 3 d'une part et entre les axes de pivotement sagittal X1 et frontal X2 d'autre part.

Par « biellette », on comprendra ici une pièce rigide non déformable dans les conditions d'utilisation normales de l'orthèse 1. Les liaisons pivot 31-37 raccordées par une biellette 38-45 donnée sont donc reliées entre elles de manière fixe de sorte que la distance entre leurs axes de rotation respectifs est constante en conditions normales d'utilisation.

La liaison mécanique de hanche 4 ainsi obtenue est plus ergonomique, dans la mesure où les axes de pivotement sagittal X1 et frontal X2 correspondent aux axes de pivotement naturels de l'articulation de la hanche de l'utilisateur et peuvent être concourants avec ces axes de pivotement naturels. Par ailleurs, l'axe de pivotement X3 de la liaison pivot virtuelle 30 étant proche du centre de rotation de l'articulation coxo-fémorale de la hanche, les mouvements parasites susceptibles de faire avancer le bassin de l'utilisateur pendant un mouvement de marche ou d'écarter sa jambe vers l'extérieur sont fortement limités. Le débattement en rotation externe peut en outre être plus ample, ce qui confère trois vrais degrés de liberté à la liaison mécanique de hanche 4 de l'orthèse 1, permettant une marche stable à l'utilisateur (sans nécessiter de béquilles).

La réalisation de la liaison pivot virtuelle 30 grâce à des liaisons pivot 31-37 raccordées en série par des biellettes 38-45 permet de rapprocher l'axe de pivotement X3 de la liaison pivot transversale 30 du centre de rotation de l'articulation coxo-fémorale de la hanche, sans risquer de blesser l'utilisateur et en s'affranchissant du besoin de placer la liaison pivot dans le corps de l'utilisateur. En effet, la décomposition de la liaison pivot transversale 30 en une pluralité de liaisons pivot 31-37 en série permet de déporter une partie de l'articulation en dehors du volume occupé par l'utilisateur tout en positionnant l'axe de pivotement résultant de cette décomposition dans ce volume, c'est-à-dire sensiblement à proximité du premier point d'intersection 11, entre l'axe de pivotement frontal X2 et l'axe de pivotement sagittal X1.

L'axe de pivotement transversal X3 coupe le plan comprenant l'axe de pivotement frontal X2 et l'axe de pivotement sagittal X1 au niveau d'un deuxième point d'intersection l2. La liaison pivot virtuelle 30 est alors configurée de sorte que la distance d1 entre ce deuxième point d'intersection I2 et le premier point d'intersection I1 (entre les axes de pivotement sagittal X1 et frontal X2) est inférieure à 150 mm lorsque l'orthèse 1 est au repos, de préférence inférieure à 120 mm, plus préférentiellement inférieure à 100 mm, par exemple inférieure ou égale à 92 mm, typiquement de l'ordre de 70 mm. Par ailleurs, le deuxième point d'intersection I2 est situé à une distance d2 inférieure ou égale à 90 mm du plan sagittal P1 de l'orthèse 1 lorsque l'orthèse 1 est au repos, de préférence inférieure ou égale à 60 mm, plus préférentiellement inférieure ou égale à 40 mm, par exemple au moins égale à 0 mm et au plus égale à 10 mm. Le deuxième point d'intersection l2 peut alors être situé à une distance d3 inférieure ou égale à 90 mm du plan P4 (qui est normal à l'axe de pivotement frontal X2 et comprend l'axe de pivotement sagittal X1 de l'orthèse 1) lorsque l'orthèse 1 est au repos, de préférence inférieure ou égale à 70 mm. On notera que l'axe de pivotement transversal X3 est de préférence normal au plan comprenant l'axe de pivotement sagittal X1 et l'axe de pivotement frontal X2.

Dans cette configuration, l'angle de débattement possible de la liaison pivot virtuelle 30, sans que les mouvements parasites soient notables pour l'utilisateur, est au moins égal à 40° lorsque la distance d1 est de l'ordre de 70 mm, la distance d2 est comprise entre 0 mm et 10 mm et la distance d3 est inférieure ou égale à 70 mm.

L'orthèse 1 comprend en en outre des actionneurs 12, 22, 32 configurés pour actionner indépendamment la liaison pivot frontale 10, la liaison pivot transversale 30 et la liaison pivot sagittale 20. L'actionnement indépendant des différentes liaisons pivot 10, 20, 30 permet en effet de simplifier le contrôle de la liaison mécanique de hanche 4. Pour cela, l'orthèse 1 comprend un actionneur transversal 32 configuré pour actionner la liaison pivot transversale 30, un actionneur frontal 12 configuré pour actionner la liaison pivot frontale 10 et un actionneur sagittal 22 configuré pour actionner la liaison pivot sagittale 20, ces trois actionneurs 12, 22, 32 étant indépendants les uns des autres. Chaque actionneur 12, 22, 32 comprend, de manière connue en soi, un moteur configuré pour mettre en rotation un arbre de sortie 15, 24 auquel est raccordée (directement ou indirectement) la liaison pivot 10, 20, 30 correspondante.

Par ailleurs, la liaison pivot frontale 10 est raccordée mécaniquement à la structure de hanche 2, la liaison pivot sagittale 20 est raccordée mécaniquement à la structure de jambe 3 et la liaison pivot transversale 30 est raccordée mécaniquement à la liaison pivot frontale 10 et à la liaison pivot sagittale 20. Les liaisons pivot frontale 10, transversale 30 et sagittale 20 sont donc montées en série. De plus, l'actionneur sagittal 22 est monté sur la liaison pivot transversale 30 et l'actionneur transversal 32 est monté sur la liaison pivot frontale 10. Cette configuration permet ainsi l'utilisation de butées mécaniques 19a-b, 25a-b, 52 indépendantes pour chaque axe de pivotement X1, X2, X3 de la liaison mécanique de hanche 4, ce qui garantit la sécurité du patient en toutes situations car ces butées mécaniques 19a-b, 25a-b, 52 empêchent l'orthèse 1 d'effectuer des mouvements allant au-delà des débattements des articulations de l'utilisateur, même lors d'une perte de contrôle des actionneurs 12, 22, 32.

La liaison pivot transversale 30 peut notamment comprendre :
- deux premières liaisons pivot 31, 32 raccordées entre elles par une première biellette 38 ;
- une deuxième et une troisième liaison pivot 33, 34 raccordées chacune à une première liaison pivot 31, 32 correspondante par une deuxième et une troisième biellette 39, 40, respectivement, la deuxième et la troisième biellette 39, 40 étant de même longueur ;
- une quatrième liaison pivot 35 raccordée à la deuxième liaison pivot 33 par une quatrième biellette 41 qui est encastrée avec la deuxième biellette 39 ;
- une cinquième et une sixième liaison pivot 36, 37 raccordées respectivement à la deuxième liaison pivot 33 et à la quatrième liaison pivot 35 par une cinquième et une sixième biellette 42, 43, respectivement, la cinquième et la sixième biellette 42, 43 étant de même longueur ;
- une septième biellette 44 raccordant la deuxième et la troisième liaison pivot 33, 34 et étant encastrée avec la cinquième biellette 42 ; et
- une huitième biellette 45 raccordant la cinquième et la sixième liaison pivot 36, 37.

La longueur de la huitième biellette 45 est sensiblement égale à la longueur de la quatrième biellette 41 de sorte que l'écartement entre les axes de pivotement des cinquième et sixième liaisons pivot 36, 37 est sensiblement égal à l'écartement entre les axes de pivotement des deuxième et troisième liaisons pivot 33, 34. De même, la longueur de la première biellette 38 est sensiblement égale à la longueur de la septième biellette 44 de sorte que l'écartement entre les axes de pivotement des premières liaisons pivot 31, 32 est sensiblement égal à l'écartement entre les axes de pivotement des deuxième et troisième liaisons pivot 33, 34.

Lorsque l'orthèse 1 est au repos, les axes de pivotement des deuxième, troisième et cinquième liaison pivot 33, 34, 37 sont alignés. De même, les axes de pivotement de la première liaison pivot 31 qui est raccordée à la deuxième biellette 39 et des deuxième et quatrième liaisons pivot 33, 35 sont alignés.

Dans une forme de réalisation, chaque liaison pivot 31-37 et au moins une partie des biellettes 39-43 sont doublées afin d'augmenter la rigidité de la liaison pivot transversale 30, en particulier vis-à-vis des actions du sol. Pour cela, chaque liaison pivot 31-37 comprend une liaison pivot supérieure et d'une liaison pivot inférieure (voir figures 1 et 3 à 5) dont les axes de pivotement sont confondus et qui sont raccordées deux à deux par une pluralité de biellettes verticales 46. Les liaisons pivot 31-37 supérieures sont en outre raccordées deux à deux par des biellettes 38-45 supérieures et les liaisons pivot 31-37 inférieures sont raccordées deux à deux par des biellettes 39-43 inférieures. On notera que le nombre de biellettes 38-45 supérieures et 39-43 inférieures peut être différent.

La liaison pivot transversale 30 étant montée sur la liaison pivot frontale 10, le couple et la vitesse de déplacement de la liaison pivot transversale 30 dépendent de la position de ladite liaison 30.

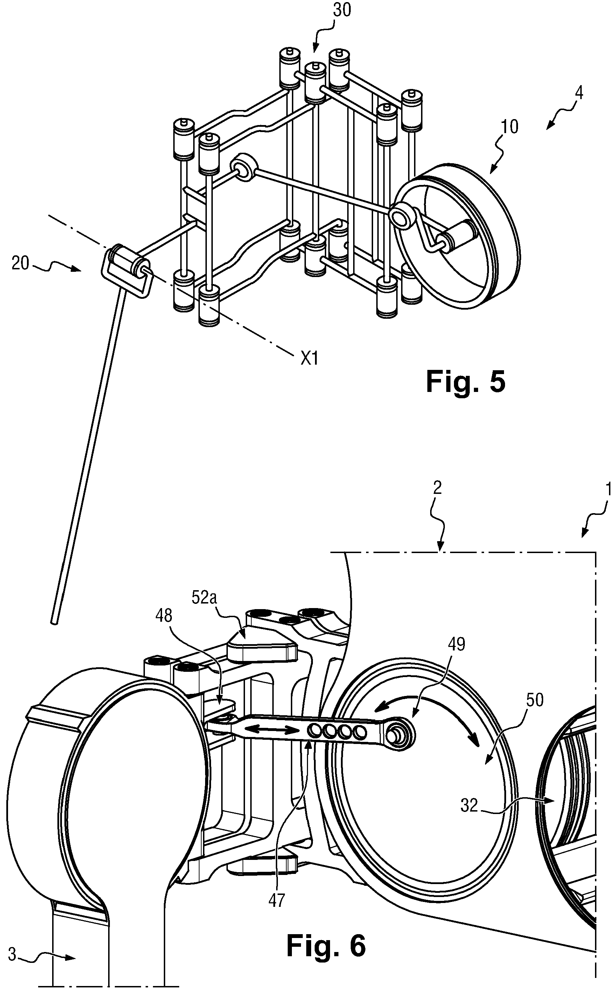

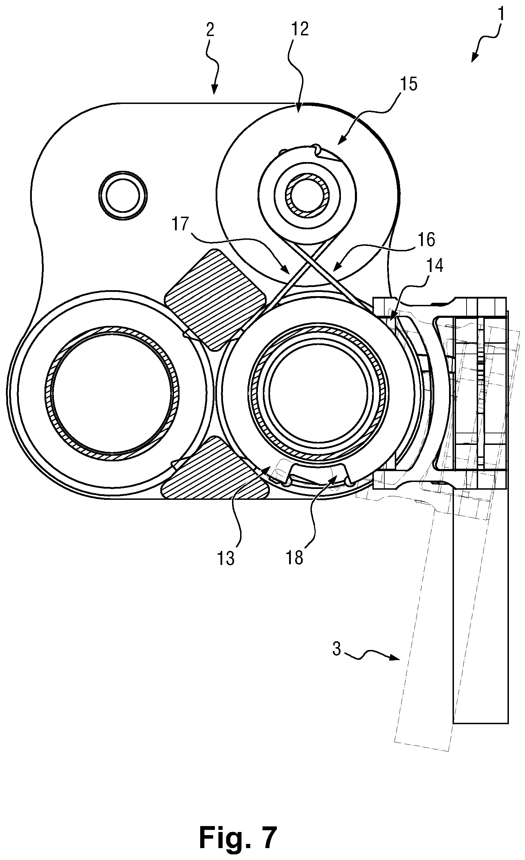

La liaison pivot frontale 10 peut par exemple comprendre une poulie 13 montée dans la structure de hanche 2 et centrée sur l'axe de pivotement frontal X2 qui est raccordée fixement à la structure de jambe 3 par une biellette d'actionnement frontale 14 (ci-après biellette frontale 14 - voir figures 2 et 6 notamment) de sorte que la rotation de la poulie 13 par rapport à la structure de hanche 2 entraine la rotation de la structure de jambe 3 autour de l'axe de pivotement frontal X2. La poulie 13 peut être montée sur l'arbre de sortie 15 (ou le cas échéant une poulie monobloc avec l'arbre de sortie 15) de l'actionneur frontal 12. En variante, l'arbre de sortie 15 de l'actionneur frontal 12 peut être déporté dans la structure de hanche 2 par rapport à l'axe de pivotement frontal X2 et entrainer la poulie 13 par l'intermédiaire de câbles 16, 17. Un exemple d'entrainement déporté a par exemple été illustré en figure 7. L'arbre de sortie 15 de l'actionneur frontal 12 est décalé par rapport à l'axe de pivotement frontal X2 et s'étend au-dessus de la poulie 13. Un premier câble 16 raccorde l'arbre de sortie 15 à la poulie 13 de manière à faire pivoter la poulie 13 dans un premier sens autour de l'axe de pivotement frontal X2 et un deuxième câble 17 raccorde la poulie 13 à l'arbre de sortie 15 de manière à faire pivoter la poulie 13 dans un deuxième sens autour de l'axe de pivotement X2. Les câbles 16, 17 se croisent à une position intermédiaire entre l'arbre de sortie 15 et la poulie 13 de sorte que le premier sens et le deuxième sens soient opposés.

L'actionnement déporté de la liaison pivot frontale 10 permet de réduire l'encombrement de l'actionnement de la liaison mécanique de hanche 4. L'utilisation de câbles 16, 17 permet en outre d'augmenter le rapport de réduction du moteur de l'actionneur frontal 12 tout en réduisant sa taille, ce qui est d'autant plus pertinent dans le cadre de la liaison pivot frontale 10 dans la mesure où le couple nécessaire à appliquer sur cette liaison 10 est plus important que celui requis pour les liaisons pivot transversale 30 et sagittale 20. Le rapport de réduction est défini par le diamètre de la poulie 13 et de la partie de l'arbre de sortie 15 sur laquelle sont fixés les câbles 16, 17. L'arbre de sortie 15 (ou le cas échéant la poulie monobloc avec l'arbre de sortie 15 et sur laquelle sont fixés les câbles 16, 17) peut par exemple présenter une diamètre deux fois plus petit que le diamètre de la poulie 13. Optionnellement, l'orthèse 1 comprend un système de précharge 18 de la tension des câbles 16, 17 intégré dans la structure de hanche 2, configuré pour appliquer une tension sur les câbles 16, 17 et garantir ainsi la précision de l'actionnement de la liaison pivot frontale 10. Le système de précharge 18 peut notamment comprendre un système de vis afin d'ajuster la tension appliquée à chaque câble 16, 17.

La liaison pivot transversale 30 est raccordée à la biellette frontale 14 de la liaison pivot frontale 10. La rotation pure de la structure de jambe 3 autour de l'axe de pivotement frontal X2 a donc pour effet de faire pivoter la liaison pivot transversale 30 et la liaison pivot sagittale 20 autour de l'axe de pivotement frontal X2.

L'actionneur transversal 32 peut comprendre une première biellette d'actionnement transversal 47 (ci-après, première biellette transversale 47) comportant une première extrémité raccordée à la liaison pivot transversale 30 par l'intermédiaire d'une première liaison rotule 48 et une deuxième extrémité raccordée à la structure de hanche 2 par l'intermédiaire d'une deuxième liaison rotule 49.

Dans une forme de réalisation, la structure de hanche 2 comprend une platine d'actionnement 50 montée de manière coaxiale avec l'axe de pivotement frontal X2 dans la structure de hanche 2 de manière à être mobile en rotation autour de l'axe de pivotement frontal X2. La platine 50 est cylindrique de révolution et est raccordée à l'arbre de sortie de l'actionneur transversal 32. La platine 50 peut le cas échéant être montée au sein de la poulie 13 de la liaison pivot frontale 10. La deuxième liaison rotule 49 est alors fixée sur la platine 50 en étant décalée par rapport à l'axe de pivotement frontal X2 afin de créer un effet de biellette-manivelle (voir figures 2 et 6 par exemple). De la sorte, la rotation de la platine 50 par rapport à la structure de hanche 2 a pour effet de déplacer la première biellette transversale 47 par rapport à la structure de hanche 2. La première liaison rotule 48 quant à elle est montée sur une deuxième biellette d'actionnement transversal 51 (ci-après deuxième biellette transversale 51) de la liaison pivot transversale 30, qui peut par exemple être montée sur la huitième biellette 45. Enfin, dans l'exemple de réalisation illustré sur les figures, la première biellette transversale 47 est montée sur la sortie de la biellette frontale 14 de la liaison pivot frontale 10. En d'autres termes, la liaison pivot transversale 30 est montée sur la sortie de la liaison pivot frontale 10.

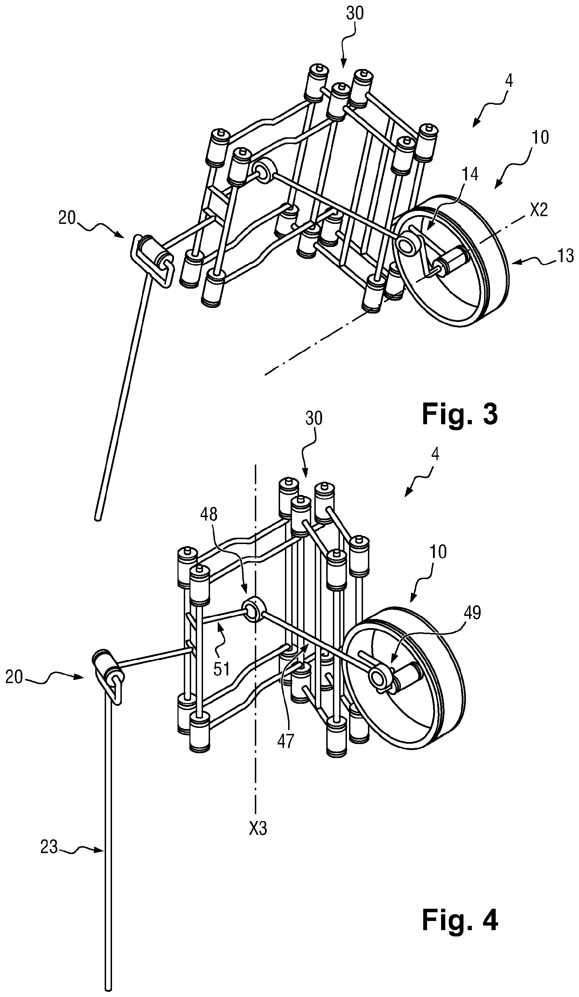

Lorsque la liaison pivot frontale 10 (et donc la biellette frontale 14) est fixe, le déplacement de la platine 50 par rapport à la structure de hanche 2 (et, le cas échéant, à la poulie 13 de la liaison pivot frontale 10) par le moteur de l'actionneur transversal 32 a pour effet de pousser ou tirer (selon le sens de rotation de la platine 50) sur la première biellette transversale 47 grâce au système de biellette-manivelle et donc de déplacer la deuxième biellette transversale 51 (voir figure 4). La liaison pivot transversale 30 tourne alors autour de son axe de pivotement X3. De plus, la liaison pivot sagittale 20 étant montée sur la sortie de la liaison pivot transversale 30, la rotation pure de la structure de jambe 3 autour de l'axe de pivotement transversal X3 a pour effet de faire pivoter la liaison pivot sagittale 20 autour de l'axe de pivotement transversal X3.

La liaison pivot sagittale 20 comprend une biellette sagittale 23 montée fixe sur l'une des biellettes 38-45, 47, 51 de la liaison pivot transversale 30, typiquement la deuxième biellette transversale 51 et/ou la huitième biellette 45. La biellette sagittale 23 peut, le cas échéant, être monolithique avec la deuxième biellette transversale 51 et/ou la huitième biellette 45. L'axe de pivotement X1 de la liaison pivot sagittale 20 est donc solidaire en mouvement d'un centre de rotation de la première liaison rotule 48.

L'arbre de sortie 24 de l'actionneur sagittal 22 peut être coaxial avec l'axe de pivotement sagittal X1 et être configuré pour mettre en rotation (directement ou indirectement, via une platine associée) la structure de jambe 3 autour de l'axe de pivotement sagittal X1. La rotation de l'arbre de sortie 24 a alors pour effet de faire tourner la structure de jambe 3 par rapport à la biellette sagittale 23 autour de l'axe de pivotement sagittal X1.

Comme indiqué ci-avant, l'orthèse 1 peut en outre comprendre des butées mécaniques 19a-b, 25a-b, 52 configurées pour limiter le débattement de la liaison mécanique de hanche 4 et protéger l'utilisateur, même en cas de panne de l'un des actionneurs 12, 22, 32. Ces butées mécaniques 19a-b, 25a-b, 25b, 52 sont indépendantes. Par ailleurs, elles sont conçues de façon à être facilement interchangeables pour permettre une personnalisation des débattements articulaires selon les capacités en débattement de chaque utilisateur.

L'orthèse 1 peut comprendre au moins l'une des butées mécaniques 19a-b, 25a-b, 25b, 52 suivantes : une butée frontale 19a-b configurée pour limiter une rotation de la structure de jambe 3 autour de l'axe de pivotement frontal X2, une butée sagittale 25a-b configurée pour limiter une rotation de la structure de jambe 3 autour de l'axe de pivotement sagittal X1, une butée transversale 52 configurée pour limiter une rotation de la structure de jambe 3 autour de l'axe de pivotement transversal X3.

La butée mécanique 19a-b de la liaison pivot frontale 10 peut notamment comprendre au moins un ergot 19a fixé sur la poulie 13 de manière à faire saillie radialement par rapport à la poulie 13, et autant de protubérances 19b fixées sur la structure de hanche 2, chaque ergot 19a étant configuré pour venir en contact avec une protubérance 19b correspondante lorsque la structure de jambe 3 atteint le débattement maximal admissible autour de l'axe de pivotement frontal X2. De préférence, l'orthèse 1 comprend une butée mécanique haute 19a-b configurée pour empêcher la rotation dans un premier sens de la liaison pivot frontale 10 au-delà d'un premier débattement maximal, par exemple + 20° par rapport à la position neutre, au repos (mouvement d'abduction de la structure de jambe 3), et une butée mécanique basse 19a-b configurée pour empêcher la rotation dans un deuxième sens de la liaison pivot frontale 10 opposé au premier sens de rotation au-delà d'un deuxième débattement maximal, par exemple - 10° par rapport à la position neutre, au repos (mouvement d'adduction de la structure de jambe 3).

La butée mécanique 52 de la liaison pivot transversale 30 peut comprendre un ergot 52a fixé sur l'une des biellettes 38-45 formant la liaison pivot transversale 30, par exemple la cinquième biellette 42, et configuré pour venir en butée contre une protubérance fixée sur ou formant au moins une autre des biellettes 38-45 de la liaison pivot transversale 30, par exemple la deuxième biellette 39 (qui est solidaire de la quatrième biellette 41). De préférence, l'orthèse 1 comprend une butée mécanique intérieure configurée pour empêcher la rotation dans un premier sens de la liaison pivot transversale 30 au-delà d'un premier débattement maximal (la protubérance correspondante étant alors fixée sur ou formée par la deuxième biellette 39), par exemple - 10° par rapport à la position neutre, au repos (mouvement de rotation interne de la structure de jambe 3), et une butée mécanique extérieure configurée pour empêcher la rotation dans un deuxième sens de la liaison pivot transversale 30 opposé au premier sens de rotation au-delà d'un deuxième débattement maximal (la protubérance correspondante étant alors fixée sur ou formée par la sixième biellette 43), par exemple + 30° par rapport à la position neutre, au repos (mouvement de rotation externe de la structure de jambe 3).

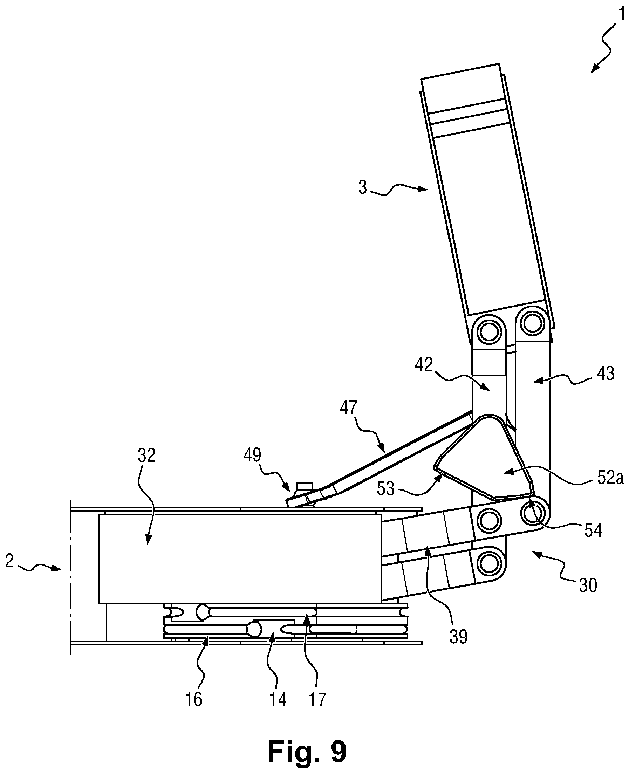

Par exemple, l'ergot 52a peut comprendre une plaque rapportée et fixée sur la cinquième biellette 42 et présentant une première face 53 formant une butée mécanique intérieure et configurée pour venir en contact avec la deuxième biellette 39 en regard, et une deuxième face 54 opposée à la première face 53, formant une butée mécanique extérieure configurée pour venir en contact avec la quatrième biellette 41 (voir figure 9 par exemple).

De préférence, lorsque les liaisons pivot 31-37 de la liaison pivot transversale 30 sont doublées, chaque butée mécanique 52 peut également être doublée de manière symétrique (haut/bas) afin d'augmenter la robustesse du système de butées. L'orthèse 1 comprend alors des butées mécaniques supérieures, fixées sur les biellettes 38-45 supérieures, et des butées mécaniques inférieures, fixées sur les biellettes 39-43 inférieures correspondantes, au droit des butées mécaniques supérieures.

La butée mécanique 25a-b de la liaison pivot sagittale 20 peut, de manière analogue à la butée mécanique de la liaison pivot frontale 10, comprendre au moins un ergot 24a solidaire en mouvement de l'arbre de sortie 24 de l'actionneur sagittal 22 de manière à faire saillie radialement par rapport à l'arbre de sortie 24, et autant de protubérances 25b montées fixes par rapport à la biellette sagittale 23, chaque ergot 25a étant configuré pour venir en contact avec une protubérance 25b correspondante lorsque la structure de jambe 3 atteint le débattement maximal admissible autour de l'axe de pivotement sagittal X1. De préférence, l'orthèse 1 comprend une butée mécanique haute 25a-b configurée pour empêcher la rotation dans un premier sens de la liaison pivot sagittale 20 au-delà d'un premier débattement maximal, par exemple + 115 ° par rapport à la position neutre, au repos (mouvement de flexion de la structure de jambe 3), et une butée mécanique basse 25a-b configurée pour empêcher la rotation dans un deuxième sens de la liaison pivot sagittal opposé au premier sens de rotation au-delà d'un deuxième débattement maximal, par exemple - 15 ° par rapport à la position neutre, au repos (mouvement d'extension de la structure de jambe 3).

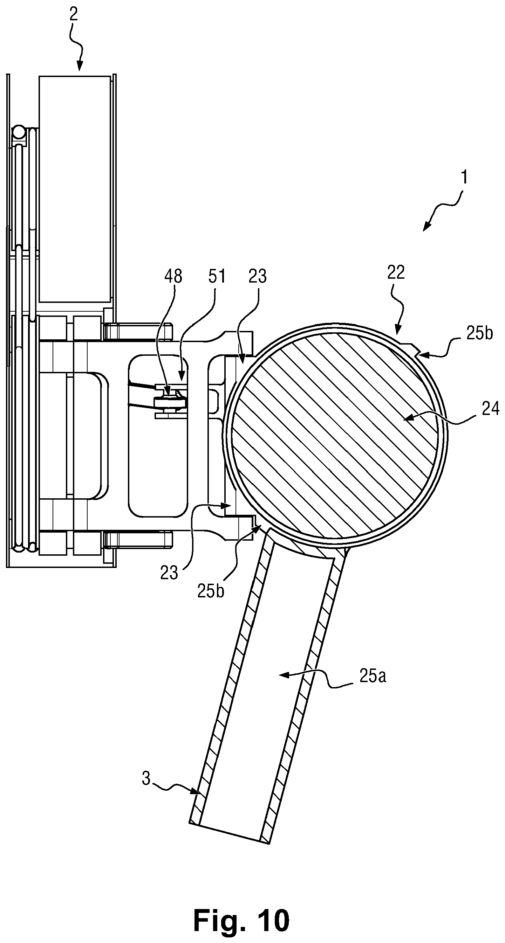

Par exemple, les ergots 24a des butées mécaniques haute et basse peuvent être formées par deux faces opposées d'une biellette de la structure de jambe 3 (voir figure 10).

## Revendications

1. Orthèse (1), par exemple un exosquelette, comprenant une liaison mécanique de hanche (4) reliant une structure de hanche (2) configurée pour se positionner en regard d'une hanche d'un utilisateur et une structure de jambe (3) configurée pour recevoir une jambe de l'utilisateur,
la liaison mécanique de hanche (4) comprenant une liaison pivot frontale (10) configurée pour autoriser un mouvement de rotation de la structure de jambe (3) par rapport à la structure de hanche (2) selon un axe de pivotement frontal (X2) de l'orthèse (1), une liaison pivot sagittale (20) configurée pour autoriser un mouvement de rotation de la structure de jambe (3) par rapport à la structure de hanche (2) selon un axe de pivotement sagittal (X1) de l'orthèse (1) et une liaison pivot transversale (30) configurée pour autoriser un mouvement de rotation de la structure de jambe (3) par rapport à la structure de hanche (2) selon un axe de pivotement transversal (X3) de l'orthèse (1) ;
l'axe de pivotement sagittal (X1) et l'axe de pivotement frontal (X2) étant concourants ;
la liaison pivot transversale (30) étant **caractérisée en ce qu'**elle est composée d'une pluralité de liaisons pivot (31-37) raccordées deux à deux par des biellettes (38-45) de sorte à former une liaison pivot virtuelle (30) de sorte que l'axe de pivotement transversal (X3) coupe le volume délimité par le plan sagittal (P1), par un plan (P4) normal à l'axe de pivotement frontal (X2) et comprenant l'axe de pivotement sagittal (X1), par la structure de hanche (2) et par la structure de jambe (3).

2. Orthèse (1) selon la revendication 1, dans laquelle l'axe de pivotement transversal (X3) est normal à un plan formé par l'axe de pivotement frontal (X2) et l'axe de pivotement sagittal (X1), un premier point d'intersection (I1) défini par l'intersection entre le plan et l'axe de pivotement transversal (X3) étant situé à une distance (d1) inférieure à 150 mm d'un deuxième point d'intersection (l2) défini par l'intersection des axes de pivotement sagittal et frontal, de préférence inférieure à 120 mm, de préférence inférieure ou égale à 100 mm, par exemple inférieure ou égale à 92 mm.

3. Orthèse (1) selon la revendication 2, dans laquelle le premier point d'intersection (I1) est situé à une distance (d2) inférieure ou égale à 90 mm du plan sagittal (P1) de l'orthèse (1), de préférence inférieure ou égal à 60 mm, plus préférentiellement inférieure ou égale à 40 mm, par exemple supérieure ou égale à 0 mm et inférieure ou égale à 10 mm.

4. Orthèse (1) selon l'une des revendications 2 et 3, dans laquelle le premier point d'intersection (l1) est situé à une distance (d3) inférieure ou égale à 90 mm d'un plan parallèle au plan frontal (P2) de l'orthèse (1) et comprenant l'axe de pivotement sagittal (X1), de préférence inférieure ou égale à 70 mm.

5. Orthèse (1) selon l'une des revendications 1 à 4, dans laquelle les liaisons pivot (31-37) composant la liaison pivot transversale (30) comprennent :
- deux premières liaisons pivot (31, 32) raccordées par une première biellette (38) ;
- une deuxième et une troisième liaison pivot (33, 34) raccordées chacune à une première liaison pivot (31, 23) correspondante par une deuxième et une troisième biellette (39, 40), respectivement, la deuxième et la troisième biellette (30, 40) étant de même longueur ;
- une quatrième liaison pivot (35) raccordée à la deuxième liaison pivot (33) par une quatrième biellette (41) qui est encastrée avec la deuxième biellette (39) ;
- une cinquième et une sixième liaison pivot (36, 37) raccordées respectivement à la deuxième liaison pivot (33) et à la quatrième liaison pivot (35) par une cinquième et une sixième biellette (42, 43), respectivement, la cinquième et la sixième biellette (42, 43) étant de même longueur ;
- une septième biellette (44) raccordant la deuxième et la troisième liaison pivot (33, 34) et étant encastrée avec la cinquième biellette (42) ; et
- une huitième biellette (45) raccordant la cinquième et la sixième liaison pivot (36, 37).

6. Orthèse (1) selon l'une des revendications 1 à 5, dans laquelle la liaison pivot frontale (10) est raccordée mécaniquement à la structure de hanche (2), la liaison pivot sagittale (20) est raccordée mécaniquement à la structure de jambe (3), et la liaison pivot transversale (30) est raccordée mécaniquement à la liaison pivot frontale (10) et à la liaison pivot sagittale (20).

7. Orthèse (1) selon l'une des revendications 1 à 6, comprenant en outre un actionneur transversal (32) configuré pour actionner la liaison pivot transversale (30), un actionneur frontal (12) configuré pour actionner la liaison pivot frontale (10) et un actionneur sagittal (22) configuré pour actionner la liaison pivot sagittale (20), l'actionneur transversal (32), l'actionneur frontal (12) et l'actionneur sagittal (22) étant indépendants les uns des autres.

8. Orthèse (1) selon l'une des revendications 1 à 7, comprenant en outre un actionneur transversal (32) configuré pour actionner la liaison pivot transversale (30), l'actionneur transversal (32) comprenant une biellette d'actionnement (47) comportant une première extrémité raccordée à la liaison pivot transversale (30) par l'intermédiaire d'une première liaison rotule (48) et une deuxième extrémité raccordée à la structure de hanche (2) par l'intermédiaire d'une deuxième liaison rotule (49).

9. Orthèse (1) selon la revendication 8, dans laquelle la deuxième liaison rotule (49) est décalée par rapport à l'axe de pivotement frontal (X2).

10. Orthèse (1) selon l'une des revendications 8 et 9, dans laquelle l'actionneur transversal (32) comprend une platine d'actionnement (50) coaxiale à l'axe de pivotement frontal (X2).

11. Orthèse (1) selon la revendication 10, dans laquelle la deuxième liaison rotule (49) est raccordée à la platine d'actionnement en étant décalée par rapport à l'axe de pivotement frontal (X2).

12. Orthèse (1) selon l'une des revendications 8 à 11, dans laquelle l'axe de pivotement sagittal (X1) de la liaison pivot sagittale (20) est solidaire en mouvement d'un centre de rotation de la première liaison rotule (48).

13. Orthèse (1) selon l'une des revendications 1 à 12, comprenant en outre un actionneur frontal (12) configuré pour actionner la liaison pivot frontale (10), l'actionneur frontal (12) étant déporté par rapport à l'axe de pivotement frontal (X2).

14. Orthèse (1) selon la revendication 13, dans laquelle l'actionneur frontal (12) comprend deux câbles (16, 17) montés croisés d'une part sur un arbre de sortie (15) de l'actionneur frontal (12) et d'autre part sur la liaison pivot frontale (10) afin de faire pivoter la liaison pivot frontale (10) autour de l'axe de pivotement frontal (X2).

15. Orthèse (1) selon l'une des revendications 1 à 14, comprenant en outre au moins l'une parmi les butées mécaniques (19a, 19b ; 25a, 25b, 52) suivantes : une butée frontale (19a, 19b) configurée pour limiter une rotation de la structure de jambe (3) autour de l'axe de pivotement frontal (X2), une butée sagittale (25a, 25b) configurée pour limiter une rotation de la structure de jambe (3) autour de l'axe de pivotement sagittal (X1), une butée transversale (52) configurée pour limiter une rotation de la structure de jambe (3) autour de l'axe de pivotement transversal (X3).

## Patentansprüche

1. Orthese (1), beispielsweise ein Exoskelett, die eine mechanische Hüftverbindung (4) umfasst, die eine Hüftstruktur (2), die ausgelegt ist, um sich einer Hüfte eines Benutzers zugewandt zu positionieren, und eine Beinstruktur (3), die ausgelegt ist, um ein Bein des Benutzers aufzunehmen, verbindet,
wobei die mechanische Hüftverbindung (4) eine frontale Schwenkverbindung (10) umfasst, die ausgelegt ist, um eine Drehbewegung der Beinstruktur (3) relativ zur Hüftstruktur (2) gemäß einer frontalen Schwenkachse (X2) der Orthese (1) zu gestatten, eine sagittale Schwenkverbindung (20), die ausgelegt ist, um eine Drehbewegung der Beinstruktur (3) relativ zur Hüftstruktur (2) gemäß einer sagittalen Schwenkachse (X1) der Orthese (1) zu gestatten, und eine transversale Schwenkverbindung (30), die ausgelegt ist, um eine Drehbewegung der Beinstruktur (3) relativ zur Hüftstruktur (2) gemäß einer transversalen Schwenkachse (X3) der Orthese (1) zu gestatten;
wobei die sagittale Schwenkachse (X1) und die frontale Schwenkachse (X2) zusammenfallen;
wobei die transversale Schwenkverbindung (30) **dadurch gekennzeichnet ist, dass** sie aus einer Vielzahl von Schwenkverbindungen (31-37) zusammengesetzt ist, die paarweise durch Stangen (38-45) derart verbunden sind, dass sie eine virtuelle Schwenkverbindung (30) bilden, so dass die transversale Schwenkachse (X3) das Volumen schneidet, das durch die Sagittalen Ebene (P1), durch eine zur frontalen Schwenkachse (X2) senkrechte Ebene (P4), die die sagittale Schwenkachse (X1) umfasst, durch die Hüftstruktur (2) und durch die Beinstruktur (3) begrenzt wird.

2. Orthese (1) nach Anspruch 1, wobei die transversale Schwenkachse (X3) senkrecht zu einer Ebene ist, die von der frontalen Schwenkachse (X2) und der sagittalen Schwenkachse (X1) gebildet wird, wobei sich ein erster Schnittpunkt (I1), der von der Schnittstelle zwischen der Ebene und der transversalen Schwenkachse (X3) definiert wird, in einem Abstand (d1) von weniger als 150 mm von einem zweiten Schnittpunkt (I2), der von der Schnittstelle der sagittalen und der frontalen Schwenkachse definiert wird, vorzugsweise weniger als 120 mm, vorzugsweise weniger als oder gleich 100 mm, beispielsweise weniger als oder gleich 92 mm, entfernt befindet.

3. Orthese (1) nach Anspruch 2, wobei sich der erste Schnittpunkt (I1) in einem Abstand (d2) von weniger als oder gleich 90 mm von der sagittalen Ebene (P1) der Orthese (1), vorzugsweise weniger als oder gleich 60 mm, noch bevorzugter weniger als oder gleich 40 mm, beispielsweise mehr als oder gleich 0 mm und weniger als oder gleich 10 mm, entfernt befindet.

4. Orthese (1) nach einem der Ansprüche 2 und 3, wobei sich der erste Schnittpunkt (I1) in einem Abstand (d3) von weniger oder gleich 90 mm von einer zur frontalen Ebene (P2) der Orthese (1) parallelen Ebene befindet und die sagittale Schwenkachse (X1) umfasst, vorzugsweise von weniger oder gleich 70 mm.

5. Orthese (1) nach einem der Ansprüche 1 bis 4, wobei die Schwenkverbindungen (31-37), die die transversale Schwenkverbindung (30) bilden, umfassen:
- zwei erste Schwenkverbindungen (31, 32), die durch eine erste Pleuelstange (38) verbunden sind;
- eine zweite und eine dritte Schwenkverbindung (33, 34), die jeweils durch eine zweite und eine dritte Pleuelstange (39, 40) mit einer entsprechenden ersten Schwenkverbindung (31, 23) verbunden sind, wobei die zweite und die dritte Pleuelstange (30, 40) gleich lang sind;
- eine vierte Schwenkverbindung (35), die durch eine vierte Pleuelstange (41), die mit der zweiten Pleuelstange (39) eingepasst ist, mit der zweiten Schwenkverbindung (33) verbunden ist;
- eine fünfte und eine sechste Schwenkverbindung (36, 37), die jeweils durch eine fünfte und eine sechste Pleuelstange (42, 43) jeweils mit der zweiten Schwenkverbindung (33) und mit der vierten Schwenkverbindung (35) verbunden sind, wobei die fünfte und die sechste Pleuelstange (42, 43) gleich lang sind;
- eine siebte Pleuelstange (44), die die zweite und die dritte Schwenkverbindung (33, 34) verbindet und mit der fünften Pleuelstange (42) eingepasst ist; und
- eine achte Pleuelstange (45), die die fünfte und die sechste Schwenkverbindung (36, 37) verbindet.

6. Orthese (1) nach einem der Ansprüche 1 bis 5, wobei die frontale Schwenkverbindung (10) mechanisch mit der Hüftstruktur (2) verbunden ist, die sagittale Schwenkverbindung (20) mechanisch mit der Beinstruktur (3) verbunden ist und die transversale Schwenkverbindung (30) mechanisch mit der frontalen Schwenkverbindung (10) und mit der sagittalen Schwenkverbindung (20) verbunden ist.

7. Orthese (1) nach einem der Ansprüche 1 bis 6, die ferner einen transversalen Aktuator (32) umfasst, der zum Betätigen der transversalen Schwenkverbindung (30) ausgelegt ist, einen frontalen Aktuator (12), der zum Betätigen der frontalen Schwenkverbindung (10) ausgelegt ist, und einen sagittalen Aktuator (22), der zum Betätigen der sagittalen Schwenkverbindung (20) ausgelegt ist, wobei der transversale Aktuator (32), der frontale Aktuator (12) und der sagittale Aktuator (22) voneinander unabhängig sind.

8. Orthese (1) nach einem der Ansprüche 1 bis 7, die ferner einen transversalen Aktuator (32) umfasst, der zum Betätigen der transversalen Schwenkverbindung (30) ausgelegt ist, wobei der transversale Aktuator (32) eine Betätigungspleuelstange (47) umfasst, die ein erstes Ende aufweist, das über eine erste Kugelgelenkverbindung (48) mit der transversalen Schwenkverbindung (30) verbunden ist, und ein zweites Ende, das über eine zweite Kugelgelenkverbindung (49) mit der Hüftstruktur (2) verbunden ist.

9. Orthese (1) nach Anspruch 8, wobei die zweite Kugelgelenkverbindung (49) relativ zur frontalen Schwenkachse (X2) versetzt ist.

10. Orthese (1) nach einem der Ansprüche 8 und 9, wobei der transversale Aktuator (32) eine Betätigungsplatte (50) umfasst, die zur frontalen Schwenkachse (X2) koaxial ist.

11. Orthese (1) nach Anspruch 10, wobei die zweite Kugelgelenkverbindung (49) mit der Betätigungsplatte verbunden ist, indem sie relativ zur frontalen Schwenkachse (X2) versetzt ist.

12. Orthese (1) nach einem der Ansprüche 8 bis 11, wobei die sagittale Schwenkachse (X1) der sagittalen Schwenkverbindung (20) bewegungsmäßig fest mit einem Rotationszentrum der ersten Kugelgelenkverbindung (48) verbunden ist.

13. Orthese (1) nach einem der Ansprüche 1 bis 12, die ferner einen frontalen Aktuator (12) umfasst, der zum Betätigen der frontalen Schwenkverbindung (10) ausgelegt ist, wobei der frontale Aktuator (12) relativ zur frontale Schwenkachse (X2) versetzt ist.

14. Orthese (1) nach Anspruch 13, wobei der frontale Aktuator (12) zwei Kabel (16, 17) umfasst, die kreuzweise einerseits an einer Abtriebswelle (15) des frontalen Aktuators (12) und andererseits an der frontalen Schwenkverbindung (10) angebracht sind, um die frontale Schwenkverbindung (10) um die frontale Schwenkachse (X2) zu schwenken.

15. Orthese (1) nach einem der Ansprüche 1 bis 14, die ferner mindestens einen von den folgenden mechanischen Anschlägen (19a, 19b; 25a, 25b, 52) umfasst: einen frontalen Anschlag (19a, 19b), der zur Begrenzung einer Rotation der Beinstruktur (3) um die frontale Schwenkachse (X2) ausgelegt ist, einen sagittalen Anschlag (25a, 25b), der zur Begrenzung einer Rotation der Beinstruktur (3) um die sagittale Schwenkachse (X1) ausgelegt ist, einen transversalen Anschlag (52), der zur Begrenzung einer Rotation der Beinstruktur (3) um die transversale Schwenkachse (X3) ausgelegt ist.

## Claims

1. An orthosis (1), for example an exoskeleton, comprising a mechanical hip link (4) connecting a hip structure (2) configured to be positioned facing a hip of a user and a leg structure (3) configured to receive a leg of the user,
the mechanical hip link (4) comprising a frontal pivot link (10) configured to allow rotation movement of the leg structure (3) relative to the hip structure (2) about a frontal pivot axis (X2) of the orthosis (1), a sagittal pivot link (20) configured to allow rotation movement of the leg structure (3) relative to the hip structure (2) about a sagittal pivot axis (X1) of the orthosis (1) and a transverse pivot link (30) configured to allow rotation movement of the leg structure (3) relative to the hip structure (2) about a transverse pivot axis (X3) of the orthosis (1);
the sagittal pivot axis (X1) and the frontal pivot axis (X2) being concurrent;
the transverse pivot link (30) being **characterized in that** it is composed of a plurality of pivot links (31-37) connected two by two by connecting rods (38-45) so as to form a virtual pivot link (30) so that the transverse pivot axis (X3) intersects the volume delimited by the sagittal plane (P1), by a plane (P4) normal to the frontal pivot axis (X2) and comprising the sagittal pivot axis (X1), by the hip structure (2) and by the leg structure (3).

2. The orthosis (1) according to claim 1, wherein the transverse pivot axis (X3) is normal to a plane formed by the frontal pivot axis (X2) and the sagittal pivot axis (X1), a first intersection point (11) defined by the intersection between the plane and the transverse pivot axis (X3) being located at a distance (d1) less than 150 mm from a second intersection point (I2) defined by the intersection of the sagittal and frontal pivot axes, preferably less than 120 mm, preferably less than or equal to 100 mm, for example less than or equal to 92 mm.

3. The orthosis (1) according to claim 2, wherein the first intersection point (11) is located at a distance (d2) less than or equal to 90 mm from the sagittal plane (P1) of the orthosis (1), preferably less than or equal to 60 mm, more preferably less than or equal to 40 mm, for example greater than or equal to 0 mm and less than or equal to 10 mm.

4. The orthosis (1) according to one of claims 2 and 3, wherein the first intersection point (11) is located at a distance (d3) less than or equal to 90 mm from a plane parallel to the frontal plane (P2) of the orthosis (1) and comprising the sagittal pivot axis (X1), preferably less than or equal to 70 mm.

5. The orthosis (1) according to one of claims 1 to 4, wherein the pivot links (31-37) composing the transverse pivot link (30) comprise:
- two first pivot links (31, 32) connected by a first connecting rod (38);
- a second and a third pivot link (33, 34) each connected to a corresponding first pivot link (31, 23) by a second and a third connecting rod (39, 40), respectively, the second and the third connecting rods (30, 40) having the same length;
- a fourth pivot link (35) connected to the second pivot link (33) by a fourth connecting rod (41) which is fixedly joined to the second connecting rod (39);
- a fifth and a sixth pivot link (36, 37) connected respectively to the second pivot link (33) and to the fourth pivot link (35) by a fifth and a sixth connecting rod (42, 43), respectively, the fifth and the sixth connecting rods (42, 43) having the same length;
- a seventh connecting rod (44) connecting the second and the third pivot link (33, 34) and being fixedly joined to the fifth connecting rod (42); and
- an eighth connecting rod (45) connecting the fifth and the sixth pivot link (36, 37).

6. The orthosis (1) according to one of claims 1 to 5, wherein the frontal pivot link (10) is mechanically connected to the hip structure (2), the sagittal pivot link (20) is mechanically connected to the leg structure (3), and the transverse pivot link (30) is mechanically connected to the frontal pivot link (10) and to the sagittal pivot link (20).

7. The orthosis (1) according to one of claims 1 to 6, also comprising a transverse actuator (32) configured to actuate the transverse pivot link (30), a frontal actuator (12) configured to actuate the frontal pivot link (10) and a sagittal actuator (22) configured to actuate the sagittal pivot link (20), the transverse actuator (32), the frontal actuator (12) and the sagittal actuator (22) being independent of one another.

8. The orthosis (1) according to one of claims 1 to 7, also comprising a transverse actuator (32) configured to actuate the transverse pivot link (30), the transverse actuator (32) comprising an actuating connecting rod (47) including a first end connected to the transverse pivot link (30) by means of a first ball joint link (48) and a second end connected to the hip structure (2) by means of a second ball joint link (49).

9. The orthosis (1) according to claim 8, wherein the second ball joint link (49) is offset relative to the frontal pivot axis (X2).

10. The orthosis (1) according to one of claims 8 and 9, wherein the transverse actuator (32) comprises an actuating turntable (50) coaxial with the frontal pivot axis (X2).

11. The orthosis (1) according to claim 10, wherein the second ball joint link (49) is connected to the actuating turntable while being offset relative to the frontal pivot axis (X2).

12. The orthosis (1) according to one of claims 8 to 11, wherein the sagittal pivot axis (X1) of the sagittal pivot link (20) moves with a center of rotation of the first ball joint link (48).

13. The orthosis (1) according to one of claims 1 to 12, also comprising a frontal actuator (12) configured to actuate the frontal pivot link (10), the frontal actuator (12) being offset relative to the frontal pivot axis (X2).

14. The orthosis (1) according to claim 13, wherein the frontal actuator (12) comprises two cables (16, 17) mounted crossing on an output shaft (15) of the frontal actuator (12) on the one hand, and on the other hand on the frontal pivot link (10) in order to pivot the frontal pivot link (10) around the frontal pivot axis (X2).

15. The orthosis (1) according to one of claims 1 to 14, also comprising at least one of the following mechanical stops (19a, 19b ; 25a, 25b, 52): a frontal stop (19a, 19b) configured to limit rotation of the leg structure (3) around the frontal pivot axis (X2), a sagittal stop (25a, 25b) configured to limit rotation of the leg structure (3) around the sagittal pivot axis (X1), a transverse stop (52) configured to limit rotation of the leg structure around the transverse pivot axis (X3).
